(19) Europäisches Patentamt
European Patent Office
Office européen des brevets

(11) Publication number: **0 200 942 B1**

(12) **EUROPEAN PATENT SPECIFICATION**

(45) Date of publication of patent specification: **25.08.93**

(51) Int. Cl.$^5$: **C07D 295/112**, A61K 31/40, A61K 31/445, A61K 31/135

(21) Application number: **86104875.9**

(22) Date of filing: **09.04.86**

The file contains technical information submitted after the application was filed and not included in this specification

(54) **New derivatives of an aminoketone.**

(30) Priority: **11.04.85 JP 75379/85**
**28.05.85 JP 113103/85**
**19.06.85 JP 131629/85**
**04.12.85 JP 271381/85**

(43) Date of publication of application:
**12.11.86 Bulletin 86/46**

(45) Publication of the grant of the patent:
**25.08.93 Bulletin 93/34**

(84) Designated Contracting States:
**DE FR GB IT NL SE**

(56) References cited:
**EP-A- 0 004 000**
**EP-A- 0 105 632**
**EP-A- 0 163 537**
**EP-A- 0 193 875**

**CHEMICAL ABSTRACTS, vol. 102, no. 17, 29 April 1985, Columbus, OH (US); p. 571, no. 148887q***

(73) Proprietor: **NIPPON KAYAKU KABUSHIKI KAISHA**
**Tokyo Fujimi Bldg. 11-2 Fujimi 1-chome**
**Chiyoda-ku**
**Tokyo 102(JP)**

(72) Inventor: **Shiozawa, Akira**
**22-7, Horisaki**
**Omiya-shi(JP)**
Inventor: **Ishikawa, Michio**
**Hasune-Famiruhaitsu 2-307 7-10, Sakashita-3-chome**
**Itabashi-ku Tokyo(JP)**
Inventor: **Izumi, Giichi**
**33-16, Daizawa-5-chome**
**Setagaya-ku Tokyo(JP)**
Inventor: **Sakitama, Katsuhiko**
**1, Kanamecho-2-chome**
**Toshima-ku Tokyo(JP)**
Inventor: **Narita, Kazuhisa**
**27-21-303, Hayamiya-4-chome**
**Nerima-ku Tokyo(JP)**
Inventor: **Kurashige, Shuji**
**771-2, Sakawa**
**Urawa-shi(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3. 10/3.6/3.3. 1)

(74) Representative: **Türk, Dietmar, Dr. rer. nat.**
**Türk, Gille, Hrabal, Leifert Patentanwälte**
**Brucknerstrasse 20**
**D-40593 Düsseldorf (DE)**

## Description

BACKGROUND OF THE INVENTION

2-Methyl-1-(4-methylphenyl)-3-piperidino-1-propanone (generic name: tolperisone) is known as one of β-aminopropiophenone derivatives having a central muscle relaxant activity (G.B. 1,213,639). Tolperisone is widely used in Japan for the clinical treatment of spastic paralysis or motor palsy resulting from muscular hypertonia.

The potency and duration of tolperisone in its clinical use, however, are not always satisfactory, and the improvements thereof have been desired.

In JP-A-59-190982 a 4'-fluoromethyl-3-amino-2-methyl substituted propiophenone derivative and its acid addition salt is described which has muscle-relaxing, spinal reflex inhibiting and anticonvulsant activities.

EP-A-105 632 describes also a propanone derivative which is useful as centrally acting muscle relaxant in the treatment of spasticity in mammals.

Furthermore, EP-A-163 537 describes 1-propanone derivatives and its physiologically acceptable acid addition salts which are useful as central muscle relaxants.

SUMMARY OF THE INVENTON

The object of this invention is to provide new aminoketone derivatives having a central muscle relaxant activity. The compounds of this invention have a strong muscle relaxant activity, keep their effect for a long time and are also low in toxicity, so that their use as an excellent muscle relaxant is expected.

The new aminoketone derivatives according to this invention are represented by the following general formula (I):

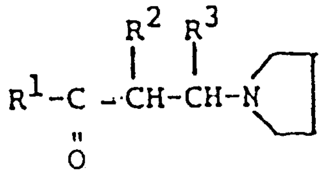

(I)

or a pharmaceutically acceptable salt thereof,
wherein $R^1$ is phenyl substituted by trihalogenmethyl or 4-($C_1$-$C_4$)alkoxy-5,6,7,8-tetrahydro-1-naphthyl, and one of $R^2$ and $R^3$ is a hydrogen atom and the other is ($C_1$-$C_4$)alkyl.

DETAILED DESCRIPTION OF THE INVENTION

In the compounds of this invention represented by the above-shown formula, the lower alkyl group, which is defined to be $C_1$-$C_4$ alkyl groups, may be selected from preferably $C_1$-$C_3$ alkyl groups, such as methyl, ethyl, n-propyl, isopropyl, n-butyl and isobutyl.

The lower alkoxy group, which are defined to be $C_1$-$C_4$ groups, may be selected from preferably $C_1$-$C_3$ alkoxyl groups such as methoxy, ethoxy, n-propoxy, isopropoxy, n-butoxy and tert-butoxy.

The halogen atom may be a chlorine, bromine or fluorine atom.

The trihalogenomethyl group may be trichloromethyl or trifluoromethyl.

Typical examples of the compounds of this invention are shown in Table I and Table II.

Table I

| Compound No. | $R^1$ | Compound No. | $R^1$ |
|---|---|---|---|
| 3-4 | 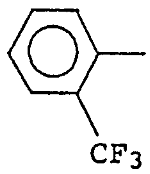 | 4-1 | 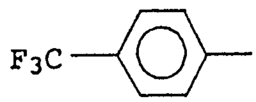 |
| 3-12 | 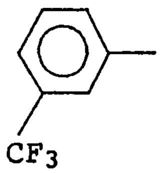 | 4-6 | 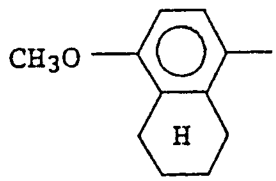 |

Table II

(Compounds represented by the general formula (I)

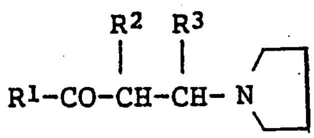 wherein $R^1$, $R^2$ and $R^3$

are defined as stated in Table II below.)

| Compound No. | $R^1$ | $R^2$ | $R^3$ |
|---|---|---|---|
| 2-1 | 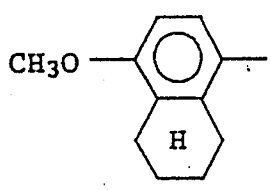 | $CH_3$ | H |
| 2-2 | 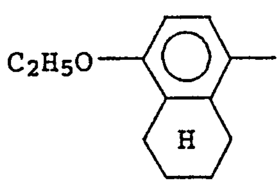 | $CH_3$ | H |
| 2-3 | 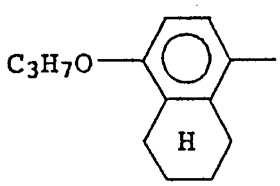 | $CH_3$ | H |

Table II (continued)

| Compound No. | $R^1$ | $R^2$ | $R^3$ |
|---|---|---|---|
| 2-11 | 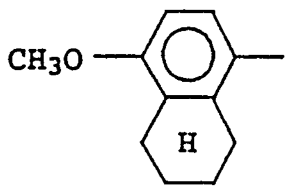 | $C_2H_5$ | H |
| 2-18 | 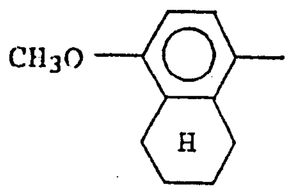 | H | $CH_3$ |

Among the compounds of this invention of which the typical examples were shown above, the preferred compounds are those of $R^1$ is a group selected from the groups of the formulae

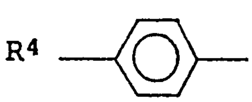

and

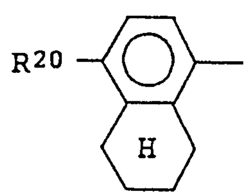

in which $R^{20}$ is a lower alkoxy group, and $R^4$ is the same as the definition described above), $R^2$ is a lower alkyl group, and $R^3$ is a hydrogen atom.

The preferred compounds, when indicated by the numbers (Compound No.) shown in the above tables, are as follows: 2-1, 2-3, 2-11, and 4-1, Especially preferred compounds are, for example, 2-1, 2-11 and 4-1, etc.

The compounds of this invention can be obtained by reacting an amine represented by the formula:

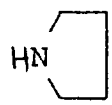

(b)

with ① a compound represented by the general formula:

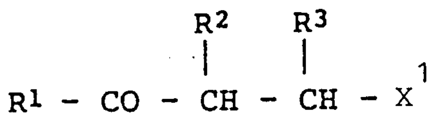

(a)

(wherein $X^1$ is halogen atom, and $R^1$, $R^2$ and $R^3$ are as defined above),
② formaldehyde and a compound represented by the general formula:

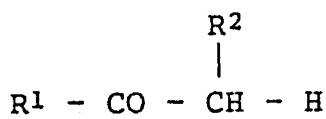

(c)

(wherein $R^1$ and $R^2$ are as defined above), or
③ a compound represented by the general formula:

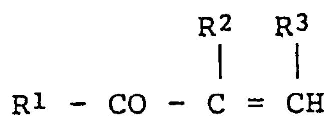

(wherein $R^1$, $R^2$ and $R^3$ are as defined above).

The reaction is preferably carried out at a temperature of 0-200 °C for a period of about 0.5-48 hours.

The process for preparing the compounds of this invention is illustrated more particularly below.

(1) In case of using the compound ①, the process comprises condensing, in an inert solvent, a compound represented by the general formula:

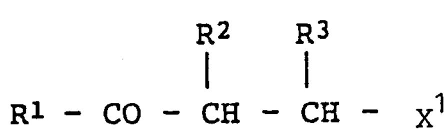

(a)

(wherein $X^1$ is halogen atom, and $R^1$, $R^2$ and $R^3$ are as defined above) and the amine pyrrolidine .

This condensation reaction is preferably carried out in an inert solvent, for example, dimethylformamide, a ketone such as acetone or an alcohol such as ethanol, by adding a base catalyst such as anhydrous potassium carbonate and potassium iodide, at a temperature of from 0 ° to 200 °C, preferably from 10 °C to near the boiling point of the solvent, for a period of about 0.5 to 48 hours. In the reaction, pyrrolidine or its salt is used in an amount of 0.5 equivalent or more, preferably 1 to 10 equivalents, to one equivalent of the halogen compound of the formula (a).

A typical example of the compounds of the formula (a) is 1-(4-methoxy-5,6,7,8-tetrahydro-1-naphthyl)-4-chloro-1-butane.

(2) The process using the compound ② is suited for obtaining the compounds of the formula (I) in which $R^3$ is a hydrogen atom, that is, new aminoketone derivatives represented by the general formula:

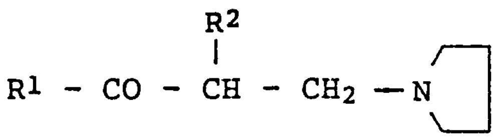

(I-a)

In this case, the process comprises reacting a compound represented by the general formula (c), with formaldehyde and pyrrolidine.

As for the ratio of the compound of the formula (c), formaldehyde and the amine pyrrolidine used in the reaction, formaldehyde is used in a ratio of usually 0.5 equivalent or more, preferably 1 to 10 equivalents, more preferably 1.5 to 6 equivalents, to one equivalent of the compound of the formula (c), and the amine or its salt is used in a ratio of usually 0.5 to 10 equivalents, preferably 1 to 3 eqivalents to one equivalent of the compound of the formula (c). Formaldehyde can be used as in the form of formalin or paraformaldehyde. The reaction can be carried out under the Mannich reaction conditions, preferably in the presence of a catalytic amount of an acid, especially hydrochloric acid. Although the reaction can be accomplished without using solvent, it is preferred to use an alcohol solvent such as propanol, isopropanol, butanol or isobutanol, a ketone solvent such as acetone or methyl ethyl ketone or an ester solvent such as ethyl acetate.

The reaction is preferably carried out at a temperature from 0° to 200°C, preferably from 10°C to near the boiling point of the solvent used, for a period of about 0.5 to 48 hours.

Listed below are the examples of the compounds of the formula (c) used in the reaction: 1-(2-α,α,α-trifluoromethylphenyl)-1-propanone, 1-(3-α,α,α-trifluoromethylphenyl)-1-propanone, 1-(4-α,α,α-trifluoromethylphenyl)-1-propanone,

1-(4-methoxy-1-naphthyl)-1-propanone,

(3) The process using the compound ③ is suited for obtaining the componds of the formula (I)

that is, the compounds represented by the following general formula:

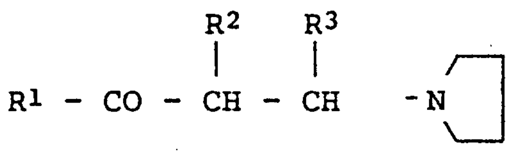

(I)

The compounds of the above formula can be readily obtained by reacting an amine of formula (b) with a compound represented by the following general formula:

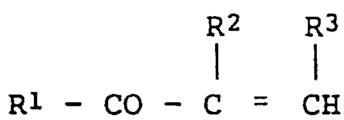

(d)

(wherein $R^1$, $R^2$ and $R^3$ are as defined above)under the Michael reaction conditions.

The ratio of the compound of the formula (d) and an amine used in the reaction is usually 0.5 equivalent or more, preferably 1 to 10 equivalents of amine to one equivalent of the compound of the formula (d).

Although the reaction can be accomplished without solvent, it is better to use a solvent. Alcohols such as methanol and ethanol can be used as solvent. For obtaining a desirable result, the reaction is carried out at a temperature from 0° to 200°C, preferably from 0°C to near the boiling point of the solvent used, for a period of 0.5 to 48 hours.

Listed below is an example of the compounds of the formula (d) used in the reaction: 1-(4-methoxy-5,6,7,8-tetrahydro-1-naphthyl)-2-butene-1-one.

The compounds of the formulae (a), (c) and (d) used as starting compound in this reaction can be obtained usually by reacting a compound represented by the general formula:

$R^1$-H (e)

(wherein $R^1$ is as defined above)
and a compound represented by the general formula:

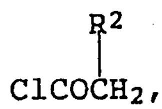, 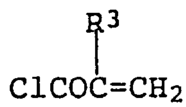 or 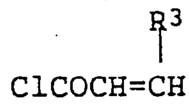

(wherein $R^2$ and $R^3$ are as defined above).

The compounds of the formula (c) can be obtained by the following method: that is, the Grignard reagent or the lithium reagent is prepared by reacting magnesium or lithium with a halogenated benzene derivative or a halogenated tetrahydronaphthalene derivative. These reagents are reacted with the corresponding aldehydes to give alcohols. The alcohols are oxidized with for example chromic anhydride.

In addition, when the substituent on the benzene or tetrahydronaphthyl group in the general formula (I) is a halogen atom such as chlorine atom, the desired compound can be obtained by the Sandmeyer or Schiemann reaction of the amine derivatives such as

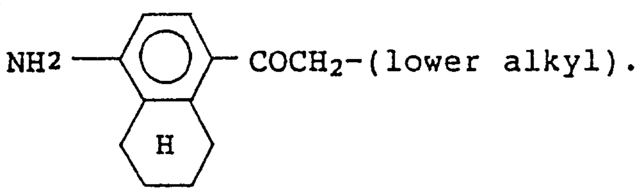.

The compounds of this invention represented by the general formula (I) are purified and isolated from the reaction solution in the usual way. Such compounds can be obtained in the form of free bases or these salts by properly selecting the reaction conditions or the treating process.

Free bases, if desired, can be transformed into acid addition salts by a conventional method. Such acid addition salts include inorganic acid salts such as hydrochloride, hydrobromide, sulfate and phosphate and organic acid salts such as formate, acetate, citrate, maleate, fumarate, tartrate, lactate and methanesulfonate.

It is to be noted that some of the compounds of this invention have one asymmetric carbon atom in the molecule and therefore there exist theoretically, two optical isomers, so that the present invention comprehends the racemates and optical isomers of these compounds. Optically active compounds can be obtained from the racemates by a known method. For example,a diastereomer salt of the desired free base was prepared with the optically active acids, and then the optical isomer was isolated.

In use of the obtained compounds of this invention as a central muscle relaxant, the compounds can be administered either orally or parenterally. The effective dose varies depending on the condition and age of the patient being treated and the method of administration, but it is usually 0.1-20mg/kg/day.

The compounds of this invention are administered in the form of pharmaceutical preparations composed by mixing the compounds with an appropriate pharmaceutical carrier. Such pharmaceutical preparations include tablets, granules, fine grains, powders, capsules, injections and suppositories.

EFFECT

The pharmaceutical activity of the compounds of this invention is described below.

1. <u>Spinal reflex (flexor reflex) inhibitory action</u>

Animals (male rats; Wistar strain) were anesthetized with urethane and α-chloralose, and their tibial nerve was dissected, and stimulated (0.1 msec, 0.1 Hz, ultra-maximum stimulation) by the stimulator (Model MSE-3; Nippon Koden). The evoked electromyogram recorded through a needle electrode inserted into the ipsilateral muscle tibialis was amplified and displayed on a cathode-ray oscilloscope. The amplitude of this evoked electromyogram was recorded on a pen recorder through a peak holder.The activity of the compounds was expressed by flexor reflex inhibition rate. That is, the flexor reflex inhibition rate was

calculated from the following equation (A):

$$\text{Inhibition rate} = \frac{A - B}{A} \times 100\ (\%) \qquad (A)$$

wherein A is the average amplitude of the electromyogram in the period of 10 minuts before the administration of the test compound, and B is the average amplitude of the electromyogram in the period of 30 minutes after the intravenous (i.v.) administration of 5 mg/kg of the test compound dissolved in a physiological saline solution.

The results are shown in Table III below.

Table III

| Compound No. | Inhibition rate (%) |
|---|---|
| 4-1 | 40.3 |
| 2-1 | 53.6 |
| 2-11 | 50.0 |

2. Action to ischemic decerebrate rigidity of rat

The effects on the ischemic decerebrate rigidity were investigated by using Fukuda et al method (H. Fukuda, T. Ito, S. Hashimoto and Y. Kudo: Japan. J. Pharmacol., 24, 810 (1974)). The rigidity is due to hyperactivity of α-motorneurones. The rigid animals provide a good experimental model for some type of spasticity in man.

METHOD:

A tracheal cannula was inserted in each of male Wistar rat under ether anesthesia. Both of common carotid arteries were ligated and the basilar artery was cauterized with a bipolar coagulator to block the blood circulation and to prepare a rigidity sample. The rigidity was recorded as described below. A rat was fixed on its back on a fixing stand and its forepaws were allowed to grisp an end of a celluloid plate provided with strain gauges on both sides thereof. A change of the resistance observed when the celluloid plate was forced up by the rigidity of the forepaws was recorded as a tension through a bridge circuit on a self-balancing recorder. A rigidity inhibition rate was calculated according to the following equation:

$$\text{Rigidity inhibition rate} = \frac{D - C}{D} \times 100\ (\%)$$

when C represents an average tension (g) for 10 minutes at peak period before the administration of a test compound and D represents an average tension (g) for 10 minutes at peak period recorded after the intravenous administration 3.5 mg/kg of a test compound.

The results are shown in Table IV.

Table IV

| Compound No. | Inhibition rate (%) |
|---|---|
| 2-1 | 46.1 |
| 2-11 | 53.9 |
| 4-1 | 23.6 |

3. Acute toxicity

$LD_{50}$ (mg/kg) of the each test compound given to mice intravenously was determined.
The results are shown below.

Table V

| Compound No. | $LD_{50}$ (Mice; i.v.) mg/kg |
|---|---|
| 4-1 | 61.1 |
| 2-1 | 38.1 |
| 2-11 | 35.4 |

As viewed above, the compounds of this invention have an excellent inhibitory action on spinal reflex (flexor reflex) and are also low in toxicity. Further, the compounds of this invention show a depression activity on the decerebrate rigidity which is regarded as one of the good experimental models of spastic palsy, and are long-acting. They also have an anticonvulsive action. Thus, it is expected that the compounds of this invention would be of use as a central muscle relaxant having an excellent therapeutical effect on spastic palsy resulting from for example cerebral vascular trouble, cerebral paralysis and spondylosis and on muscular hypertonia accompanied with various diseases.

The process for producing the compounds of this invention will now be described with reference to Examples.

Example 1

1-(4-Methoxy-5,6,7,8-tetrahydro-1-naphthyl)-2-ethyl-3-pyrrolidino-1-propanone (Compd. Nos. 2-11) hydrochloride.

A mixture of 1-(4-methoxy-5,6,7,8-tetrahydro-1-naphthyl)-1-butanone (2.50g), paraformaldehyde (0.42g), pyrrolidine hydrochloride (1.51g), and hydrochloric acid (0.2 mℓ) in isopropyl alcohol (7 mℓ) was refluxed for 15 hrs. The reaction mixture was evaporated in vacuo to give the residue. The residue was partitioned into water and ethyl ether. The aqueous layer, was extracted, neutralized with ammonia water, and then extracted with ethyl ether. The organic layer was dried over anhydrous magnesium sulfate. The mixture was filtered and the filtrate was evaporated in vacuo to give 1-(4-methoxy-5,6,7,8-tetrahydro-1-naphthyl)-2-ethyl-3-pyrrolidino-1-propanone (2.13 g, Yield: 62.8 %) as an oil.

$$IR \ \nu_{max}^{neat} :$$

1625 $cm^{-1}$
NMR δ ($CDC\ell_3$, TMS): 0.90 (t, 3H, J = 7.0 Hz; $CH_2CH_3$), 1.2 - 2.1 (m, 10H,

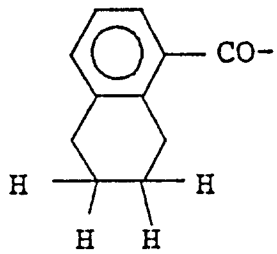,

$-CH_2CH_3$, and

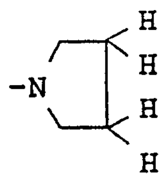),

2.2 - 3.1 (m, 10H;

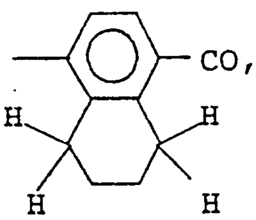

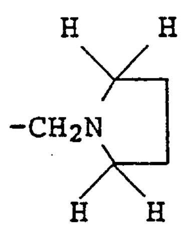),

3.1 - 3.7 (m, 1H,

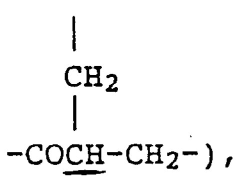

6.70 (d, 1H, J = 8.0 Hz;

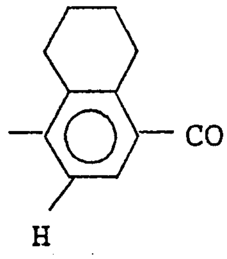 ),

7.56 (d, 1H, J=8.0 Hz;

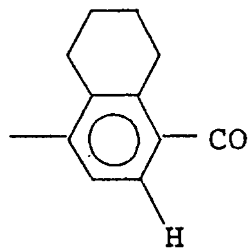 )

Mass m/z (relative intensity): 315 ($M^+$, 3.7), 244 (48.0), 229 (74.2), 215 (35.8), 189 (47.2), 84 (100).

The free base was dissolved in ethyl ether, reacted by introducing dry hydrochloride gas, and then filtered off. The crude crystals were recrystallized from $CH_2C\ell_2$-AcOEt to give 2.14 g of the HCℓ salts as colorless prisms having a melting point of 164 - 165 °C.

In the same way, there can be obtained, for example, the compounds of Table VII by using inter alia the starting compounds of Table VI. (Compound Nos. in Table VI indicate Nos. of the compounds of this invention obtained by using the starting compounds shown in the table below).

Table VI-2

($R^1$ and $R^2$ substituents of Compounds represented by the formula 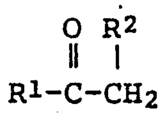 and pyrrolidine

which are used as starting materials for the compounds represented by the general formula (I) listed in Table II.)

| Compound No. | $R^1$ | $R^2$ |
|---|---|---|
| 2-1 | 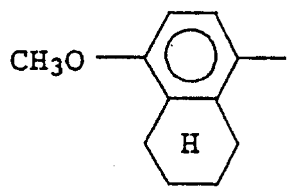 | $CH_3$ |
| 2-2 | 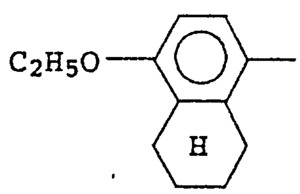 | $CH_3$ |
| 2-3 | 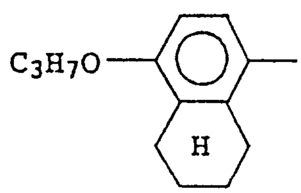 | $CH_3$ |

Table VI-2 (continued)

| Compound No. | $R^1$ | $R^2$ |
|---|---|---|
| 2-11 | 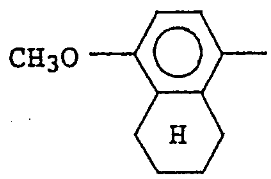 | $C_2H_5$ |
| 2-18 | 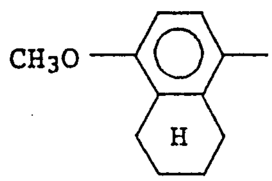 | H |

Table VII

| Compound No. | Yield % | Melting Point of HCl Salt | Solvent for Recrystallization |
|---|---|---|---|
| 3-4 | 19.2 | 148-149 | (MeOH Acetone) |
| 3-12 | 32.9 | 144-145 | (Acetone $Et_2O$) |
| 4-1 | 44.8 | | |
| 2-1 | 83.9 | 150-151 | ($CH_2Cl_2$ AcOEt) |
| 2-3 | 86.6 | 161-162 | ($CH_2Cl_2$ AcOEt) |
| 2-11 | 62.8 | 164-165 | ($CH_2Cl_2$ AcOEt) |

Example 2

1-(4-Trifluoromethylphenyl)-2-methyl-3-pyrrolidino-1-propanone (Compd. No. 4-1) hydrochloride.

A mixture of 1-(4-α,α,α-trifluoromethylphenyl)-1-propanone (2.50g), paraformaldehyde (1.10g), pyrrolidine hydrochloride (1.60g), and hydrochloric acid (0.1 mℓ) was refluxed for 16 hours. The mixture was evaporated in vacuo to give the residue. The residue was partitioned into water and ethyl acetate. The aqueous layer was neutralized with ammonia water and then extracted with ethyl acetate. The organic layer was dried over anhydrous magnesium sulfate and then filtered. The filtrate was evaporated in vaccuo to give 1-(4-trifluoromethylphenyl)-2-methyl-3-pyrrolidino-1-propanone (1.58g; yield: 44.8%) as an oil.

IR $\nu_{max}^{neat}$ :

1690 $cm^{-1}$

NMR δ ($CDCℓ_3$, TMS): 1.25 (d, 3H, J = 7.0 Hz; $-COCH(CH_3)CH_2-$), 1.4 - 2.1 (m, 4H,

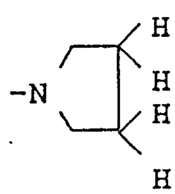

),

2.3 - 3.2 (m, 6H,

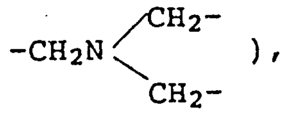

3.65 (1H, m;

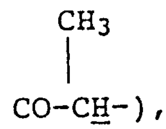

7.70 (2H, d, J = 8.0 Hz, aromatic H), 8.05 (2H, d, J = 8.0 Hz, aromatic H).
Mass m/z (relative intensity): 285 (2.21, $M^+$), 214 (100); 173 (100), 145 (100), 95 (29.7), 84 (100).

The free base was dissolved in ethyl ether, reacted by introducing dry hydrochloride gas, and then filtered off. The crude crystals were recrystallized to give the HCℓ salts as colorless prisms (1.43g),

Example 3

1-(4-Methoxy-5,6,7,8-tetrahydro-1-naphthyl)-2-methyl-3-pyrrolidino-1-propanone (compd. No. 2-1) hydrochloride.

A mixture of 1-(4-methoxy-5,6,7,8-tetrahydro-1-naphthyl)-1-propanone (4.37g), paraformaldehyde (1.80g), pyrrolidine hydrochloride (3.23g), and hydrochloric acid (0.2 mℓ) in isopropyl alcohol (50 mℓ) was refluxed for 8 hours. The reaction mixture was evaporated in vacuo to give the residue. The residue was partitioned into water and ethyl ether. The aqueous layer was extracted, neutralized with ammonia water, and then extracted with ethyl ether. The organic layer was dried over anhydrous magnesium sulfate and then filtered. The filtrate was evaporated in vacuo to give 1-(4-methoxy-5,6,7,8-tetrahydro-1-naphthyl)-2-methyl-3-pyrrolidino-1-propanone. (5.06g; yield: 83.9%).

$$IR\ \nu_{max}^{neat}\ :$$

1690 $cm^{-1}$
NMR δ ($CDCℓ_3$, TMS): 1.20 (d, 3H, J = 7.0 Hz; $-COCH(\underline{CH_3})CH_2-$), 1.4 - 2.1 (m, 8H,

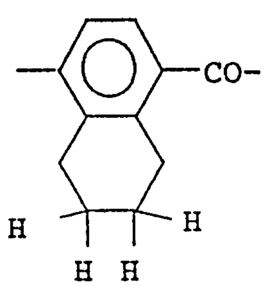

and

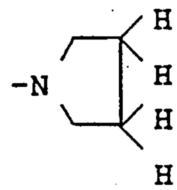

),

2.1 - 3.2 (m, 10H,

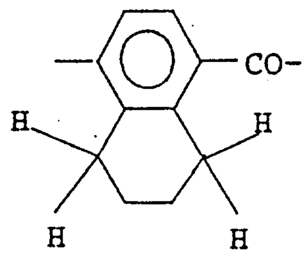

and

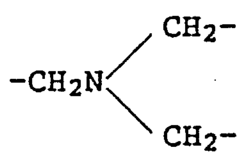

),

3.86 (s,3H,

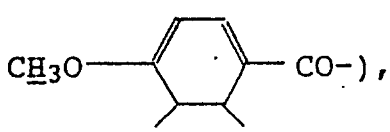

6.70 (d, 1H, J = 8.0 Hz;

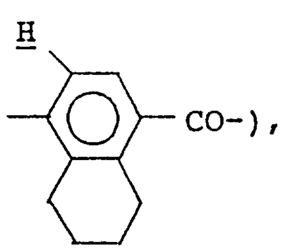

7.53 (d, 1H, J = 8.0 Hz;

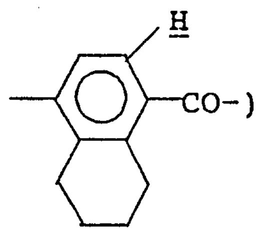

Mass m/z (relative intensity): 301 ($M^+$, 4.87), 230 (35.3), 215 (24.3), 189 (19.3), 187 (10.3), 84 (100).

The free base was dissolved in ethyl ether, reacted by introducing dry hydrochloride gas, and then filtered off. The crude crystals were recrystallized from $CH_2C\ell_2$ - AcOEt to give the HCℓ salts (3.60g) as colorless prisms, m.p. 150 - 151 °C.

By using the compounds of the formulae

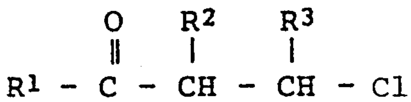

and

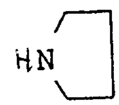

as starting materials (the combinations of $R^1$, $R^2$, $R^3$, being the same as in Tables I and II) and by following the procedures of the above examples, there can be obtained all of the compounds of this invention represented by the general formula (I). Examples of the combinations of $R^1$, $R^2$, and $R^3$ in said two starting compounds are shown in Table VIII. (Compound Nos. in the table correspond to those in Table VI).

Table VIII

| Compound No. | $R^1$ | $R^2$ | $R^3$ |
|---|---|---|---|
| 4-1 | 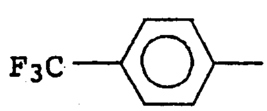 | H | $CH_3$ |

Table II (continued)

| Compound No. | $R^1$ | $R^2$ | $R^3$ |
|---|---|---|---|
| 2-1 | 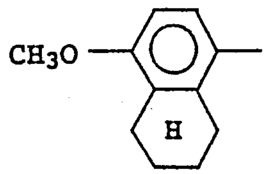 | $CH_3$ | H |
| 2-11 | 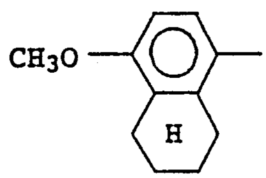 | $C_2H_5$ | H |
| 2-18 | 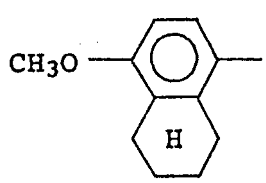 | H | $CH_3$ |

Example 4

1-(4-Methoxy-5,6,7,8-tetrahydro-1-naphthyl)-3-methyl-3-pyrrolidino-1-propanone (Compd. No. 2-18) hydrochloride.

A solution of 1-(4-methoxy-5,6,7,8-tetrahydro-1-naphthyl)-2-butene-1-one (3.0g) and pyrrolidine (1.85g) in ethanol (100 mℓ) was stirred for 3 hours at room temperature. The mixture was evaporated in vacuo to give the residue. The residue was partitioned into water and ethyl ether. The ethyl ether was washed with water. The organic layer was extracted with 2N hydrochloric acid. The aqueous layer was neutralized with ammonia water and then extracted with ethyl ether. The ethyl ether was dried over anhydrous magnesium sulfate and then filtered. The filtrate was evaporated in vacuo to give 1-(4-methoxy-5,6,7,8-tetrahydro-1-naphthyl)-3-methyl-3-pyrrolidino-1-propanone (2.31g, Yield: 53.6%) as an oil.

$$IR\ \nu_{max}^{neat} :$$

1680 $cm^{-1}$
NMR δ ($CDC\ell_3$, TMS): 1.11 (d, 3H, J = 7.0 Hz; $-CH_3$), 1.5-2.1 (m, 8H,

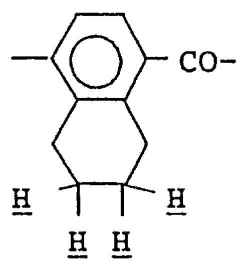

and

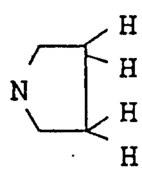 ),

2.3 - 2.8 (m, 8H,

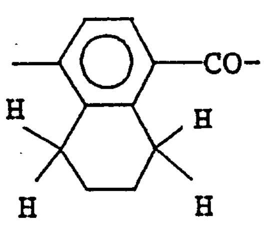

and

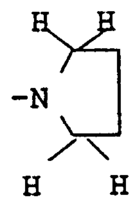

),

2.8 - 3.2 (m, 3H, -COC$\underline{H}_2$C$\underline{H}$-), 3.86 (s, 3H, $-OCH_3$), 6.70 (d, 1H, J = 8.0 Hz,

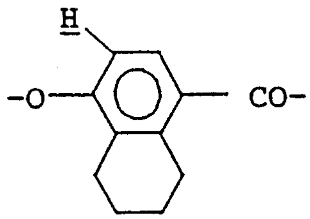

),

7.60 (d, 1H, J = 8.0 Hz,

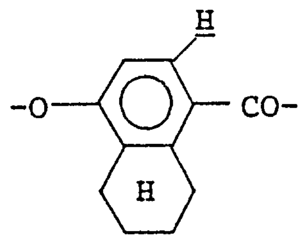

)

Mass m/z (relative intensity): 301 ($M^+$, 5.6), 230 (21.5), 215 (33.0), 189 (21.4), 187 (14.7), 98 (100), 97 (30.7).

The free base was dissolved in ethyl ether, reacted by introducing dry hydrochloride gas, and then filtered off. The crude crystals were recrystallized from $CH_2C\ell_2$ - AcOEt to give the $HC\ell$ salts as colorless prisms, m.p. 149 - 150 °C.

By using the compounds of the formulae

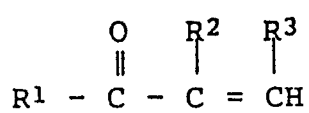

and pyrrolidine as starting materials (the combinations of $R^1$, $R^2$, and $R^3$ being the same as in Tables I and II) and by following the procedures of the above example, there can be obtained the compounds of this invention represented by the general formula (I)

Hydrochlorides of these compounds can be obtained by making the reaction solution acidic with dilute hydrochloric acid and then the reaction mixture was evaporated <u>in vacuo</u> to give the residue. The residue was dissolved in water and then washed with ethyl acetate. The aqueous layer was extracted with dichloromethane and the extract was then dried over anhydrous magnesium sulfate. The mixture was filtered and then the filtrate was evaporated <u>in vacuo</u> to give the residue. Examples of the compounds obtained by this method are shown in Table IX.

Table IX

| Compound No. | Yield % | Melting Point of HCl Salt | Solvent for Recrystallization |
|---|---|---|---|
| 4-18 | 92.2 | 123-125 | (Acetone) |

Reference Example 1

4-Methoxy -5,6,7,8-tetrahydro-1-propionaphthone

A solution prepared by adding dropwise 4.27 g of propionic acid chloride to a soltion of 6.16 g of anhydrous aluminum chloride in 70 mℓ of dichloromethane under ice cooling and stirring the mixture for 30 minutes was added dropwise to a solution of 6.24 g of 1-methoxy -5,6,7 8-tetrahydronaphthalene in 20 mℓ of dichloromethane and further stirred at room temperture for 3 hours. The reaction solution was poured into ice water and extracted with dichloromethane. The dichloromethane layer was dried over anhydrous magnesium sulfate. The drying agent was filtered out and the filtrate was concentrated under reduced pressure to obtain 4-methoxy -5,6,7,8-tetrahydro-1-propionaphthone in a yield of 7.94 g (94.5%).

$$IR\ \nu_{max}^{neat}:$$

1670 $cm^{-1}$

Reference Example 2

1-(4-Methoxy-5,6,7,8-tetrahydro-1-naphthyl)-2-butenyl-1-one

A solution prepared by adding dropwise 3.87 g of crotonoyl chloride to a solution of 4.94 g of anhydrous aluminum chloride in 50 mℓ of dichloromethane under ice cooling and stirring the mixture for 30 minutes was added dropwise to a solution of 5 g of 1-methoxy-5,6,7,8-tetrahydronaphthalene in 20 mℓ of dichloromethane and further stirred at room temperature for 3 hours. The resulting reaction solution was poured into ice water and extracted with dichloromethane. The dichloromethane layer was dried over anhydrous magnesiuum sulfate. The drying agent was filtered out and the filtrate was concentrated under reduced pressure to obtain 6:95 g (98.0% yield) of 1-(4-methoxy-5,6,7,8-tetrahydro-1-naphthyl)-2-butenyl-1-one as an oily substance.

$$IR\ \nu_{max}^{neat}:$$

1660 $cm^{-1}$

Reference Example 3

1-(5,6,7,8-Tetrahydro-1-naphthyl)-1-propanone

0.963 g of propionealdehyde was added dropwise at room temperature to a Grignard solution prepared by adding 1-bromo-5,6,7,8-tetrahydronaphthalene in an amount of 1.75 g to 242 mg of magnesim in 20 mℓ of anhydrous ether, and the resulting solution was stirred at room temperature for one hour. The reaction solution was added with a saturated aqueous solution of ammonium chloride for decomposition and then extracted with ether. This ether layer was dried over anhydrous magnesim sulfate and filtered, and the filtrate was concentrated under reduced pressure to obtain 1.40 g of 1-(5,6,7,8-tetrahydro-1-naphthyl)-1-propanol as a crude oily product.

This product was dissolved in 20 mℓ of acetone, then added with a solution consisting of 72.5 mℓ of water and 4.72 g of chromic anhydride under ice cooling and stirred at room temperature for one hour. The

reaction solution was concentrated under reduced pressure and, after distilling off acetone, extracted with ethyl acetate. The ethyl acetate layer was dried over anhydrous magnesium sulfate and filtered to remove the drying agent, and the filtrate was concentrated under reduced pressure to obtain 640 mg of 1-(5,6,7,8-tetrahydro-1-naphthyl)-1-propanone as an oily substance.

$$IR\ \nu_{max}^{neat} :$$

1690 $cm^{-1}$

**Claims**

1. An aminoketone derivative represented by the following general formula:

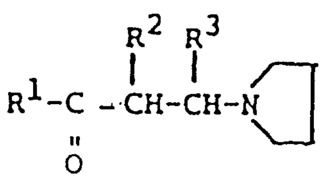

(I)

or a pharmaceutically acceptable salt thereof,
wherein $R^1$ is phenyl substituted by trihalogenmethyl or 4-($C_1$-$C_4$)alkoxy-5,6,7,8-tetrahydro-1-naphthyl, and one of $R^2$ and $R^3$ is a hydrogen atom and the other is ($C_1$-$C_4$)alkyl.

2. An aminoketone derivative according to claim 1 or a pharmaceutical acceptable salt thereof wherein $R^1$ is a phenyl substituted in para-position by trihalogenmethyl, $R^2$ is ($C_1$-$C_4$) alkyl and $R^3$ is hydrogen.

3. An aminoketone derivative or a pharmaceutically acceptable salt thereof according to claim 1, wherein the compound of the general formula (I) is 1-(4-methoxy-5,6,7,8-tetrahydro-1-naphthyl)-2-ethyl-3-pyrrolidino-1-propanone.

4. An aminoketone derivative or a pharmaceutically acceptable salt thereof according to claim 1, wherein the compound of the general formula (I) is 1-(4-methoxy-5,6,7,8-tetrahydro-1-naphthyl)-2-methyl-3-pyrrolidino-1-propanone.

5. An aminoketone derivative or a pharmaceutically acceptable salt thereof according to claim 2, wherein $R^2$ is methyl or ethyl.

6. An aminoketone derivative or a pharmaceutically acceptable salt thereof according to claim 2, wherein the compound is 1-(4-trifluoromethylphenyl)-2-methyl-3-pyrrolidino-1-propanone.

7. An aminoketone derivative or a pharmaceutically acceptable salt of any of claims 1 - 6 for use as a medicament.

8. An aminoketone derivative or a pharmaceutically acceptable salt of any of claims 1 - 6 for use in the treatment of spastic paralysis or the improvement of hypertonia of muscles.

9. A pharmaceutical composition containing aminoketone derivatives or pharmaceutically acceptable salts thereof according to any of claims 1 - 6 as active ingredient in association with a pharmaceutically acceptable, substantially non-toxic carrier or excipient.

## Patentansprüche

1. Ein durch die folgende allgemeine Formel dargestelltes Aminoketonderivat

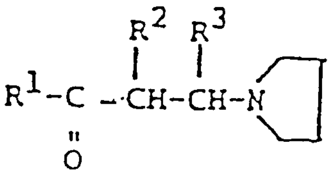

(I)

oder ein pharmazeutisch verträgliches Salz davon,
worin $R^1$ durch Trihalogenmethyl oder 4-($C_1$-$C_4$)Alkoxy-5,6,7,8-tetrahydro-1-naphthyl substiuiertes Phenyl und einer von $R^2$ und $R^3$ Wasserstoff und der andere ($C_1$-$C_4$)Alkyl ist.

2. Ein Aminoketonderivat nach Anspruch 1 oder ein pharmazeutisch verträgliches Salz davon, worin $R^1$ ein in para-Position durch Trihalogenmethyl substituiertes Phenyl, $R^2$ ($C_1$-$C_4$)Alkyl und $R^3$ Wasserstoff ist.

3. Ein Aminoketonderivat oder ein pharmazeutisch verträgliches Salz davon nach Anspruch 1, worin die Verbindung, der allgemeinen Formel (I) 1-(4-Methoxy-5,6,7,8-tetrahydro-1-naphthyl)-2-ethyl-3-pyrrolidino-1-propanon ist.

4. Ein Aminoketonderivat oder ein pharmazeutisch verträgliches Salz davon nach Anspruch 1, worin die Verbindung der allgemeinen Formel (I) 1-(4-Methoxy-5,6,7,8-tetrahydro-1-naphthyl)-2-methyl-3-pyrrolidino-1-propanon ist.

5. Ein Aminoketonderivat oder ein pharmazeutisch verträgliches Salz davon nach Anspruch 2, worin $R^2$ Methyl oder Ethyl ist.

6. Ein Aminoketonderivat oder ein pharmazeutisch verträgliches Salz davon nach Anspruch 2, worin die Verbindung 1-(4-trifluoromethylphenyl)-2-methyl-3-pyrrolidino-1-propanon ist.

7. Ein Aminoketonderivat oder ein pharmazeutisch verträgliches Salz davon nach Ansprüchen 1 bis 6 zur Verwendung als Medikament.

8. Ein Aminoketonderivat oder ein pharmazeutisch verträgliches Salz davon nach Ansprüchen 1 bis 6 zur Verwendung in der Behandlung von spastischer Paralyse oder bei der Verbesserung der Muskelhypertonie.

9. Eine pharmazeutische Zusammensetzung, enthaltend als aktiven Bestandteil Aminoketonderivate oder pharmazeutisch verträgliche Salze davon nach Ansprüchen 1 bis 6 in Verbindung mit einem pharmazeutisch verträglichen, im wesentlichen ungiftigen Träger oder Vehikel.

## Revendications

1. Dérivé d'aminocétone représenté par la formule générale :

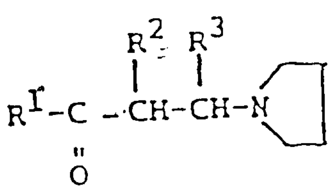

(I)

(ou un sel pharmaceutiquement acceptable de celui-ci),
dans laquelle $R^1$ est un groupe phényle substitué par un groupe trihalogénométhyle, ou 4-(alcoxy en

$C_1$-$C_4$)-5,6,7,8-tétrahydro-1-naphtyle, et l'un de $R^2$ et $R^3$ est un atome d'hydrogène et l'autre est un groupe alkyle en $C_1$-$C_4$.

**2.** Dérivé d'aminocétone ou sel pharmaceutiquement acceptable de celui-ci selon la revendication 1, dans lequel $R^1$ est un groupe phényle substitué en position ***para*** par un groupe trihalogénométhyle, $R^2$ est un groupe alkyle en $C_1$-$C_4$ et $R^3$ est l'hydrogène.

**3.** Dérivé d'aminocétone ou sel pharmaceutiquement acceptable de celui-ci selon la revendication 1, dans lequel le composé de formule générale (I) est la 1-(4-méthoxy-5,6,7,8-tétrahydro-1-naphtyl)-2-éthyl-3-pyrrolidino-1-propanone.

**4.** Dérivé d'aminocétone ou sel pharmaceutiquement acceptable de celui-ci selon la revendication 1, dans lequel le composé de formule générale (I) est la 1-(4-méthoxy-5,6,7,8-tétrahydro-1-naphtyl)-2-méthyl-3-pyrrolidino-1-propanone.

**5.** Dérivé d'aminocétone ou sel pharmaceutiquement acceptable de celui-ci selon la revendication 2, dans lequel $R^2$ est un groupe méthyle ou éthyle.

**6.** Dérivé d'aminocétone ou sel pharmaceutiquement acceptable de celui-ci selon la revendication 2, dans lequel le composé est la 1-(4-trifluorométhylphényl)-2-méthyl-3-pyrrolidino-1-propanone.

**7.** Dérivé d'aminocétone ou sel pharmaceutiquement acceptable selon l'une des revendications 1 à 6, pour son utilisation comme médicament.

**8.** Dérivé d'aminocétone ou sel pharmaceutiquement acceptable selon l'une quelconque des revendications 1 à 6, pour son utilisation dans le traitement de la paralysie spastique ou l'amélioration de l'hypertonie de muscles.

**9.** Composition pharmaceutique contenant des dérivés d'aminocétone ou des sels pharmaceutiquement acceptables de ceux-ci selon l'une quelconque des revendications 1 à 6 comme ingrédient actif en association avec un support ou excipient sensiblement non toxique, pharmaceutiquement acceptable.